(19) **Europäisches Patentamt**
**European Patent Office**
**Office européen des brevets**

(11) **EP 1 800 638 B1**

(12) **EUROPEAN PATENT SPECIFICATION**

(45) Date of publication and mention
of the grant of the patent:
**26.05.2010 Bulletin 2010/21**

(51) Int Cl.:
**A61F 13/15** *(2006.01)*          **D21H 21/22** *(2006.01)*

(21) Application number: **06126478.4**

(22) Date of filing: **19.12.2006**

(54) **Absorbent sheet and absorbent article using the same**

Absorbierende Lage und absorbierender Artikel, der diese benutzt

Feuille absorbante et article absorbant utilisant cette feuille

(84) Designated Contracting States:
**DE ES FR GB SE**

(30) Priority: **20.12.2005 JP 2005366449**

(43) Date of publication of application:
**27.06.2007 Bulletin 2007/26**

(73) Proprietor: **KAO CORPORATION**
**Chuo-ku**
**Tokyo 103-8210 (JP)**

(72) Inventors:
• **Kimura, Mika**
**c/o Kao Corporation, Research Lab.**
**Tochigi (JP)**

• **Nagahara, Shinsuke**
**Kao Corporation, Research Lab.**
**Tochigi (JP)**
• **Kimura, Mayumi**
**c/o Kao Corporation, Research Lab.**
**Tochigi (JP)**

(74) Representative: **Vossius & Partner**
**Siebertstraße 4**
**81675 München (DE)**

(56) References cited:
**EP-A- 0 359 615      EP-A- 0 889 151**
**EP-A1- 0 212 618     EP-A1- 0 528 248**
**EP-A1- 0 719 531     EP-A2- 1 023 884**
**US-A- 6 086 950**

**Description**

The present invention relates to an absorbent sheet and an absorbent article using the absorbent sheet.

Absorbent articles such as sanitary napkins are required to prevent over-hydration of skin or stuffiness, and various proposals have been made to meet the requirement. Wearers often feel discomfort after body fluid discharge due to the stuffiness or stickiness caused by the absorbed body fluid. The discomfort is particularly conspicuous after a large amount of a body fluid is excreted or after an absorbent article is worn for an extended period of time.

Such stuffiness and stickiness seem to be because, for one thing, a body fluid absorbed by an absorbent article is not immobilized in an absorbent member inside the article, such as an absorbent sheet, and rewets the skin facing side of the article and, for another, the body fluid vaporizes toward the skin by the body heat or the outside heat.

Commonly assigned JP 7-132126A discloses an absorbent article containing a moisture absorbing agent and having a moisture permeable backsheet with specific moisture permeability. The absorbent article is proposed as having a moisture absorptivity above a certain level. According to the disclosure, the absorbent article sufficiently manifests the effect of preventing over-hydration because water vapor generated from a liquid absorbed by an absorbent member is positively captured by the moisture absorbing agent. However, the absorbent article is not designed to control the generation of water vapor from liquid absorbed by the absorbent member.

Commonly assigned JP 8-246395A discloses an absorbent sheet of paper having superabsorbent polymer particles dispersed therein. The superabsorbent polymer particles are fixedly bonded to the hydrophilic fibers making the absorbent sheet. Securely fixed within the sheet, the superabsorbent polymer particles fall off the sheet hardly at all. Moreover, because the superabsorbent polymer particles undergo little gel blocking, the absorbent sheet is superior in absorption rate and liquid fixing capability.

Such a superabsorbent polymer is more capable of fixing liquid than hydrophilic fiber such as pulp. Therefore, the absorbent sheet of JP 8-246395A suppresses water vaporization. However, a body fluid once absorbed by superabsorbent polymer particles partly releases from the particles in an adsorption equilibrium state. The released liquid can generate water vapor, or the body fluid is directly released in the form of water vapor. In the case where the superabsorbent polymer particles are disposed near the skin facing surface of an absorbent article, the released water vapor can cause over-hydration.

The present invention provides an absorbent sheet containing a fibrous material and a particulate superabsorbent polymer. The particulate superabsorbent polymer is dispersed in the thickness direction of the absorbent sheet in a state of being held by the fibrous material. When the portion in the thickness direction of the absorbent sheet where the superabsorbent polymer is dispersed is divided into regions by at least one plane perpendicular to the thickness direction of the sheet, the average particle size of the superabsorbent polymer dispersed in each region decreases from the region that is the nearest to one side to the region that is the nearest to the other side of the absorbent sheet.

The present invention also provides an absorbent article having a topsheet, a backsheet, and the above-described absorbent sheet between the topsheet and the backsheet.

Fig. 1 is a schematic cross-sectional view of an absorbent sheet incorporating a first embodiment of the present invention.

Fig. 2 is a fragmentary cross-section of a preferred structure of an absorbent sheet incorporating the first embodiment.

Fig. 3A, Fig. 3B, and Fig. 3C are schematic illustrations showing a preferred process of producing an absorbent sheet according to the first embodiment, in which Fig. 3A is a wet papermaking step, Fig. 3B is a polymer sprinkling step, and Fig. 3C is a laminating step.

Fig. 4A and 4B each schematically illustrate the state of an absorbent sheet in the process shown in Figs. 3A, 3B, and 3C.

Fig. 5 is a schematic cross-sectional view of an absorbent sheet incorporating a second embodiment of the present invention.

Fig. 6 shows a preferred process of producing the absorbent sheet of the second embodiment.

Fig. 7 is a schematic cross-sectional view of an absorbent sheet incorporating a third embodiment of the present invention.

Fig. 8A and Fig. 8B are schematic illustrations showing a preferred process of producing the absorbent sheet of the third embodiment.

Fig. 9 is a schematic cross-sectional view of an absorbent sheet incorporating a fourth embodiment of the present invention.

Fig. 10 shows a preferred process of producing the absorbent sheet of the fourth embodiment.

Fig. 11 presents a perspective of a sanitary napkin as an embodiment of the absorbent article according to the invention, with a part cut away.

The present invention relates to an absorbent sheet and an absorbent article using the same which hardly cause stuffiness and gives comfort to a wearer even after a large amount of a body fluid is discharged or after the absorbent article is worn for an extended period of time as well as exhibit high absorptivity and excellent resistance to leakage.

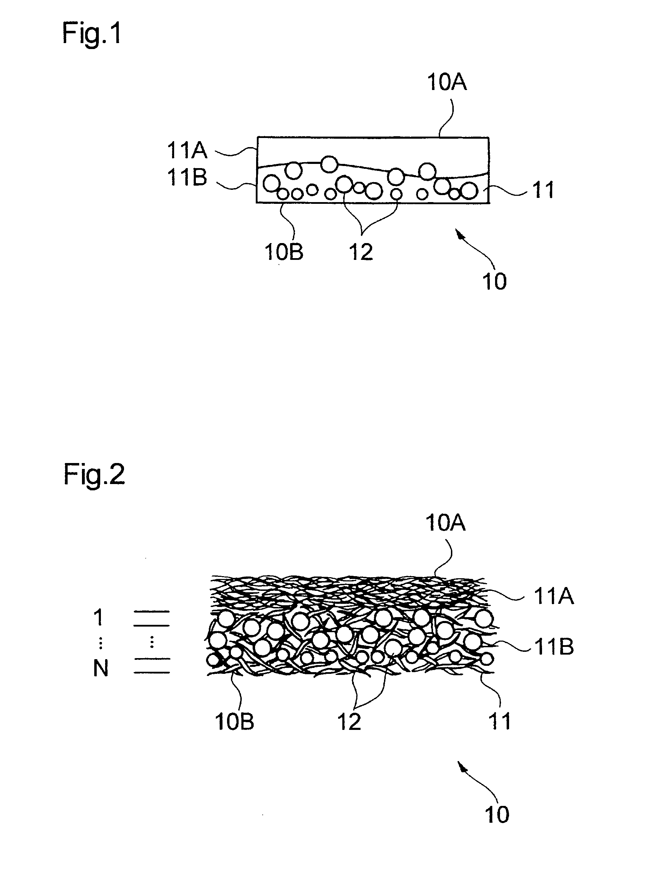

A first preferred embodiment of the absorbent sheet according to the present invention will be described with reference to Figs. 1 and 2.

As illustrated in Fig. 1, an absorbent sheet 10 of the first embodiment is a thin sheet having a first surface 10A and a second surface 10B. The absorbent sheet 10 contains a fibrous material 11 and a particulate superabsorbent polymer (polymer particles) 12. The superabsorbent polymer particles 12 are dispersed in the thickness direction of the absorbent sheet 10 in a state of being held by the fibrous material 11.

When the portion in the thickness direction of the absorbent sheet 10 where the superabsorbent polymer particles are dispersed is divided into regions by at least one plane perpendicular to the thickness direction of the sheet, the average particle size of the superabsorbent polymer 12 dispersed in each region decreases from the side of the first surface 10A to the side of the second surface 10B of the absorbent sheet.

The absorbent sheet 10 of the first embodiment preferably has two layers 11A and 11B of the fibrous material 11 as illustrated in Fig. 2. Specifically, the absorbent sheet 10 has a laminate structure in which the upper fiber layer 11A is superposed on the lower fiber layer 11B. The upper fiber layer 11A and the lower fiber layer 11B are integral with each other.

The upper fiber layer 11A has the first surface 10A that serves as an absorbing surface and preferably contains substantially no polymer particles 12. The expression "contain substantially no polymer particles" as used herein means that the polymer particles 12 are not intentionally incorporated into the upper fiber layer 11A. Existence of a trace amount of the polymer 12 in the upper fiber layer 11A that has been unintentionally incorporated in the manufacture of the absorbent sheet 10 is acceptable. While the absorbent sheet 10 is capable of absorbing liquid from either of the first surface 10A or the second surface 10B, it is preferred to use the first surface 10A as a skin facing side (the main absorbing surface) so as to prevent stuffiness in application to absorbent articles such as sanitary napkins.

The polymer particles 12 in the absorbent sheet 10 of the first embodiment will further be described. The polymer particles 12 are held by the fibrous material 11 and embeddedly supported throughout the thickness of the lower fiber layer 11B as shown in Figs. 1 and 2. The polymer particles 12 are dispersed throughout the thickness of the lower fiber layer 11B. In other words, the polymer particles 12 are three-dimensionally distributed inside the lower fiber layer 11B. The polymer particles 12 are held by the fibrous material 11 of the lower fiber layer 11B through the entanglement and by their own tackiness developed upon liquid absorption. Being so held, the polymer particles 12 are secured inside the lower fiber layer 11B and effectively prevented from falling off. Accordingly, the absorbent sheet 10 of the first embodiment is allowed to contain a large quantity of the polymer particles 12. For example, the absorbent sheet 10 of the first embodiment is capable of containing 15% to 70% by weight, preferably 25% to 65% by weight, of the polymer particles 12 based on its total weight.

When the lower fiber layer 11B is divided into regions by at least one plane perpendicular to the thickness direction, the average particle size of the superabsorbent polymer 12 dispersed in each region decreases in the direction from the region that is the nearest to the first surface 10A to the region that is the nearest to the second surface 10B. In more detail, when the lower fiber layer 11B is divided into N equal regions at planes perpendicular to its thickness direction as shown in Fig. 2, the polymer particles 12 in the first region that is the nearest to the first surface 10A have a larger average particle size than those in the Nth region that is the nearest to the second surface 10B.

It should be noted nevertheless that the polymer particles 12 present in each region have a size distribution so that each region contains polymer particles of various sizes. In some cases, the first region can contain polymer particles smaller than the average particle size of the polymer in the Nth region. In other cases, the Nth region can contain polymer particles greater than the average particle size of the polymer in the first region.

Such polymer particles disperse system in the lower fiber layer 11B can be implemented, for example, as follows.

Polymer particles 12 having a certain size distribution are sprinkled on one side of a lower fiber layer 11B that is in a dry state. The lower fiber layer 11B with the polymer particles 12 on is vibrated, whereupon the polymer particles move down through the interstices between fibers of the fibrous material 11. Smaller particles move down more easily, and greater particles are more stuck behind. As a result, there is created a polymer particle size distribution in the thickness direction such that greater particles are nearer to the side of the first surface 10A and that smaller particles are nearer to the side of the second surface 10B.

As used herein, the terms "greater particles" and "smaller particles" are relative terms that are used in size comparison to each other.

The average particle size of the polymer particles 12 in the lower fiber layer 11B is measured, for example, as follows.

The lower fiber layer 11B is divided into N (usually 3 to about 5) equal regions by at least one plane perpendicular to the thickness direction under electron microscopic observation. The average diameter of ten polymer particles randomly chosen from a region is taken as the average particle size of that region. In a case where the lower fiber layer 11B has a multi-layer structure composed of two or more sublayers, it is divided into N equal regions using the border between the adjoining sublayers as a plane at which it is divided. Where the multi-layer structure is borderless, or the border is vague, the lower fiber layer 11B is divided at equal intervals in the thickness direction.

The particulate polymer 12 can have various shapes including spheres, blocks, bales, and irregular shapes depending on the production process. The particle size of a non-spherical polymer particle is calculated as a sphere-equivalent diameter from the maximum cross-sectional area defined by the contour of the particle.

When the lower fiber layer 11B is divided into, for example, 3 equal regions, in the thickness direction, it is preferred that the polymer particles 12 in the region nearest to the first surface 10A have an average particle size of 250 to 500 $\mu$m, more preferably 250 to 350 $\mu$m, and that the polymer particles 12 in the region nearest to the second surface 10B have an average particle size of 100 to 300 $\mu$m, more preferably 100 to 250$\mu$m. When the lower fiber layer 11B is similarly divided into 3 equal regions, it is also preferred that the proportion of the polymer particles 12 present in the region nearest to the first surface 10A be 20% to 50% by weight, more preferably 30% to 40% by weight, based on the total amount of the polymer particles 12 contained in the absorbent sheet 10 and that the proportion of the polymer particles 12 in the region nearest to the second surface 10B be 30% to 70% by weight, more preferably 40% to 50% by weight, on the same basis. The weight of the polymer particles 12 as referred to here is the weight of the particles in a dry state.

The term "dry state" of the polymer 12 as referred to above and of the fibrous material 11 is intended to mean not only a state completely free of water but a practically dry state as with the case when the absorbent sheet 10 is stored under an ordinary temperature and humidity condition to reach equilibrium moisture content.

As stated, there are a larger number of the smaller particles 12 in the region on the side of the second surface 10B than in the other regions. The smaller polymer particles 12 have a larger specific area. The surface of the smaller polymer particles 12 is partly in contact with the fibers of the fibrous material 11 to form a vast number of interstices between the fibers and the particles. Thus, the number of the interstices increases in the direction from the first surface 10A to the second surface 10B to thereby create an ascending capillarity gradient toward the side of the second surface 10B. As a result, the liquid drawing power of the lower fiber layer 11B increases toward the second surface 10B.

The polymer particles 12 are dispersed in a plane perpendicular to the thickness direction of the lower fiber layer 11 B in the vicinity of the second surface 10B. They are evenly dispersed in a planar direction of the lower fiber layer 11B. Therefore, a liquid on the side of the first surface 10A of the lower fiber layer 11B is rapidly drawn toward the side of the second surface 10B and absorbed by the polymer particles 12 distributed in the vicinity of the second surface 10B. As a result, the liquid is fixed over the entire area of the lower fiber layer 11 B. The phrase "the vicinity of the second surface 10B" as used herein denotes the nearest region to the second surface 10B when the lower fiber layer 11 B, which is the portion in the thickness direction of the absorbent sheet where the particulate superabsorbent polymer is dispersed, is divided into, e.g., three equal portions in the thickness direction by planes perpendicular to the thickness direction.

It is preferred for the polymer particles 12 distributed in the vicinity of the second surface 10B to have a high swelling ratio on absorbing liquid to form many interstices described above. It is also preferred for the polymer particles 12 distributed in the vicinity of the second surface 10B to have a high absorption rate.

On absorbing liquid, the polymer particles 12 in the vicinity of the second surface 10B swell but hardly come into contact with each other because of their small size. Therefore, swelling of the polymer particles narrows the interstices without causing hindrance to the liquid's movement or gel blocking. As a result, liquid is drawn and diffused more easily.

On the other hand, most of the polymer particles 12 in the region nearest to the first surface 10A are larger ones, but they are fewer in number. Therefore, the swollen particles hardly come into contact with each other, does not hinder the liquid's flow, and cause no gel blocking. Furthermore, since the region nearest to the first surface 10A has a sparse distribution of the polymer particles 12, swelling is not so much as to reduce the interstices. As a result, a liquid on the side of the first surface 10A of the lower fiber layer 11B is allowed to rapidly move to the side of the second surface 10B.

It is preferred for the polymer particles 12 distributed in the region nearest to the first surface 10A to have a small swelling ratio and a small rate of absorption on contact with liquid so as to make a liquid move down swiftly toward the second surface 10B.

To help understand, the liquid absorption mechanism in the lower fiber layer 11B is explained using a model. Polymer particles 12 with various sizes are separated into larger particles disposed on the surface of the lower fiber layer 11B nearer to the first surface 10A and smaller particles disposed in the vicinity of the second surface 10B. The separation is ruled by the fiber-to-fiber distance in the lower fiber layer 11B. Accordingly, polymer particles of desired particle size can remain on the surface of the first surface side by selecting the lower fiber layer 11B. The amount of the polymer particles remaining on the surface of the first surface side is decided with consideration given to the prevention of gel blocking that is a factor of reducing absorbing performance. The size distribution and the amount to be sprinkled of the polymer particles are thus decided. A suitable distance between the polymer particles varies depending on the liquid to be absorbed. For example, in the case of a body fluid that can be absorbed in relatively large quantities, such as urine and perspiration, the polymer particles are sprinkled considering that they may swell about 150 to 300 times their initial volume. In the case of such a body fluid as menstrual blood or blood, the polymer particles are sprinkled considering that they may swell about 5 to 50 times their initial volume. In the latter case, assuming that spherical polymer with a uniform particle diameter (r) arranged on the first surface side surface of the lower fiber layer 11B in a regular lattice pattern swell 30 times the initial volume on absorbing artificial blood, the center-to-center distance of the spherical polymers that can produce the above-described effects would be the cube root of 30 multiplied by r.

Any conventional superabsorbent polymer can be used as the particulate polymer 12. Examples of suitable polymers include starch, crosslinked carboxymethyl cellulose, polyacrylic acid polymers and the salts thereof, such as a homo- or copolymer of acrylic acid or its alkali metal salt, and polyacrylic acid salt graft polymers. The polyacrylic acid salts preferably include a sodium salt. Also preferred are acrylic acid copolymers containing a comonomer unit derived from maleic acid, itaconic acid, acrylamide, 2-acrylamido-2-methylpropanesulfonic acid, 2-(meth)acryloylethanesulfonic acid, 2-hydroxyethyl (meth)acrylate, styrenesulfonic acid, etc. in such a proportion that does not impair the performance of superabsorbent acrylic acid polymers.

The fibrous material 11 making up the lower fiber layer 11B and holding the polymer particles 12 is preferably hydrophilic to secure liquid passage and absorption. Suitable hydrophilic fibrous materials include, but are not limited to, natural cellulosic fibers such as wood pulp (e.g., softwood or hardwood kraft pulp), cotton pulp, and straw pulp, regenerated cellulose fibers such as rayon and cuprammonium, hydrophilic synthetic fibers such as polyvinyl alcohol fiber and polyacrylonitrile fibers, and non-hydrophilic synthetic fibers (e.g., polyethylene fiber, polypropylene fiber, and polyester fiber) having been hydrophilized with a surface active agent. These hydrophilic fibers can be used either individually or as a combination of two or more thereof.

Cellulosic fibers are preferred of the hydrophilic fibers. Bulky cellulosic fibers, such as natural cellulose fibers and regenerated cellulose fibers, are particularly preferred. Wood pulp, especially softwood kraft pulp, is preferred from an economical viewpoint. Such bulky cellulosic fibers exhibit improved capabilities of dispersing and immobilizing the polymer particles 12. Moreover, the bulky cellulosic fibers help the polymer particles 12 be embedded, dispersed, and immobilized in three dimensions. Furthermore, the bulky cellulosic fibers are effective in suppressing gel blocking. For all these considerations, the bulky cellulosic fibers are preferably used in a proportion of 40% to 95% by weight, more preferably 50% to 80% by weight, based on the weight of the absorbent sheet 10. The terminology "bulky fibers" as used herein denotes fibers having a three-dimensional form, such as a twist, a crimp, a bend and/or a branch, or having a very large thickness (e.g., a fiber coarseness of 0.3 mg/m or more).

Of the bulky cellulosic fibers preferred are those having a fiber coarseness of 0.3 mg/m or more. Cellulosic fibers having a fiber coarseness of 0.3 mg/m or more can be accumulated while retaining their bulkiness to provide a bulky network structure in which to hold the polymer particles 12. In such a bulky network structure, a liquid can migrate at a high speed receiving little resistance. The fiber coarseness of the bulky cellulosic fiber is preferably 0.3 to 2 mg/m, more preferably 0.32 to 1 mg/m.

The terminology "fiber coarseness" means a measure used to represent the thickness of fiber with uneven thickness such as wood pulp. Measurement of fiber coarseness can be taken with, for example, a fiber length analyzer FS-200 available from Kajanni Electronics, Ltd.

Examples of the cellulosic fibers having a fiber coarseness of 0.3 mg/m or more are ALBACEL (softwood kraft pulp from Federal Paper Board Co.) and INDORAYON (softwood kraft pulp from PT Inti Indoraryon Utama).

Other examples of preferred bulky cellulosic fibers include those having a cross-section with a circularity of 0.5 to 1, preferably 0.55 to 1. Cellulosic fibers with a circularity of 0.5 to 1 cause little resistance to liquid migration to secure a high rate of liquid permeation. Circularity of fiber is measured as follows. A fiber is sliced vertically in a direction perpendicular to its length, and the cut area is microphotographed. The micrograph is analyzed on an image analyzer New Qube ver. 4.22 from Nexus to measure the circumference and the cross-sectional area of the fiber. The circularity of the fiber is calculated from the equation:

$$\text{Circularity} = 4\pi \, (\text{cross-sectional area})/(\text{circumference})^2$$

Measurements are made on randomly chosen 100 cut areas to obtain an average circularity.

As stated, wood pulp is preferred cellulosic fiber. Wood pulp, however, generally has a flattened cross-section as a result of delignification, having a circularity of less than 0.5. Such flat wood pulp fibers can be converted to fibers with a circularity of 0.5 or more by mercerization (swell).

That is, mercerized pulp fibers with a circularity of 0.5 to 1 which is obtained by mercerization of wood pulp are also preferred bulky cellulosic fibers. Commercially available mercerized pulps that can be used in the invention include FILTRANIER and POROSANIER, both from ITT Rayonier, Inc.

Cellulosic fibers having both a coarseness of 0.3 mg/m or more and a cross-sectional circularity of 0.5 to 1 are more preferred for their capability of forming a bulky network structure more easily and achieving a higher rate of liquid permeation.

It is also preferred to use heat fusible fibers that melt on heating and bond to each other as the fibrous material 11 in combination with the above-described bulky cellulose fibers. Using heat fusible fibers is effective in imparting sufficient wet strength to the lower fiber layer 11B. Examples of useful heat fusible fibers include polyolefin fibers such as polyethylene, polypropylene, and polyvinyl alcohol, polyester fibers, polyethylene-polypropylene conjugate fibers, polyethylene-polyester conjugate fibers, low-melting polyester-polyester conjugate fibers, polyvinyl alcohol-polypropylene conjugate fibers having a hydrophilic surface, and polyvinyl alcohol-polyester conjugate fibers having a hydrophilic surface. The conjugate fibers may have a sheath/core configuration or a side-by-side configuration. These heat fusible fibers can be used either individually or as a mixture of two or more thereof. The heat fusible fibers preferably have a fiber length of 2 to 60 and a fineness of 0.1 to 3 denier, more preferably 0.5 to 3 denier. The heat fusible fibers are preferably used in a proportion of 1% to 50% by weight, more preferably 3% to 30% by weight, based on the total weight of the absorbent sheet 10.

The fiber length of the fibrous material 11 is not critical in the present invention. When the absorbent sheet 10 is fabricated by, for example, a wet papermaking technique as described infra, a preferred fiber length of the fibrous material 11 is 1 to 20 mm.

The absorbent sheet 10 can further contain a polyamine epichlorohydrin resin, dialdehyde starch, carboxymethyl cellulose, etc. as a wet-strength additive.

The lower fiber layer 11B preferably weighs 10 to 40 $g/m^2$, more preferably 15 to 30 $g/m^2$, for the following reasons. With a weight of the lower fiber layer 11B of less than 10 $g/m^2$, the swollen polymer particles 12 can bulge outward and fall off the absorbent sheet 10. A weight of the lower fiber layer 11B exceeding 40 $g/m^2$ can result in too high a density, making the absorbent sheet 10 too hard to disperse and fix the polymer particles in three dimensions and to secure liquid permeation. Such a hard absorbent sheet 10 can cause discomfort to a wearer.

The upper fiber layer 11 having the first surface 10A functioning as a main absorbing surface of the absorbent sheet 10 will then be described. The fibrous material 11 making up the upper fiber layer 11A can be selected from those recited above for use in the lower fiber layer 11B.

Seeing that the upper fiber layer 11A is to be disposed on the skin facing side in applications to an absorbent article such as a sanitary napkin, it is preferred that the upper fiber layer 11A be stiffer and bulkier than the lower fiber layer 11B to have cushioning properties and liquid permeability.

Bulkier cellulosic fibers that are preferably used in the upper fiber layer 11A include crosslinked cellulose fibers having an intramolecular and/or intermolecular crosslinked structure. Such crosslinked cellulosic fibers are preferred as being capable of maintaining the bulk even in a wet state. Commercially available crosslinked cellulosic fibers include High Bulk Additive from Weyerhaeuser Paper Co.

In addition to the bulky cellulosic fibers described above, also preferred are crosslinked cellulosic fibers obtained by intramolecularly and/or intermolecularly crosslinking cellulosic fibers (e.g., pulp) having a fiber coarseness of 0.3 mg/m or more or a cross-sectional circularity of 0.5 to 1. Also preferred are crosslinked pulp fibers obtained by intramolecularly and/or intermolecularly crosslinking mercerized pulp fibers having a cross-sectional circularity of 0.5 to 1. More preferred are crosslinking pulp fibers having both a fiber coarseness of 0.3 mg/m or more and a cross-sectional circularity of 0.5 to 1. Even more preferred are those obtained by mercerizing pulp fibers having a coarseness of 0.3 mg/m or more to increase the circularity to 0.5 to 1, followed by crosslinking.

The upper fiber layer 11A preferably contains the aforementioned heat fusible fibers in a higher proportion than in the lower fiber layer 11B to have increased stiffness and bulkiness.

The upper fiber layer 11A contains substantially no polymer particles 12. Nevertheless, existence of up to about 5% to 20% of polymer particles 12 based on the total weight per unit area of the polymer particles 12 present in the lower fiber layer 11B is acceptable unless the polymer particles 12 are exposed on the first surface 10A of the absorbent sheet 10. Where a polymer particle 12 exists on the border between the upper fiber layer 11A and the lower fiber layer 11B, the particle is regarded as belonging to the lower fiber layer 11B even if a part of it is in the upper fiber layer 11A.

The absorbent sheet 10 of the present embodiment is featured by its small thickness despite the high proportion of the polymer particles 12. Specifically, with a weight ranging from 50 to 200 $g/m^2$, preferably 60 to 160 $g/m^2$, the thickness of the absorbent sheet 10 is as small as 0.3 to 2.0 mm, preferably 0.35 to 1.0 mm.

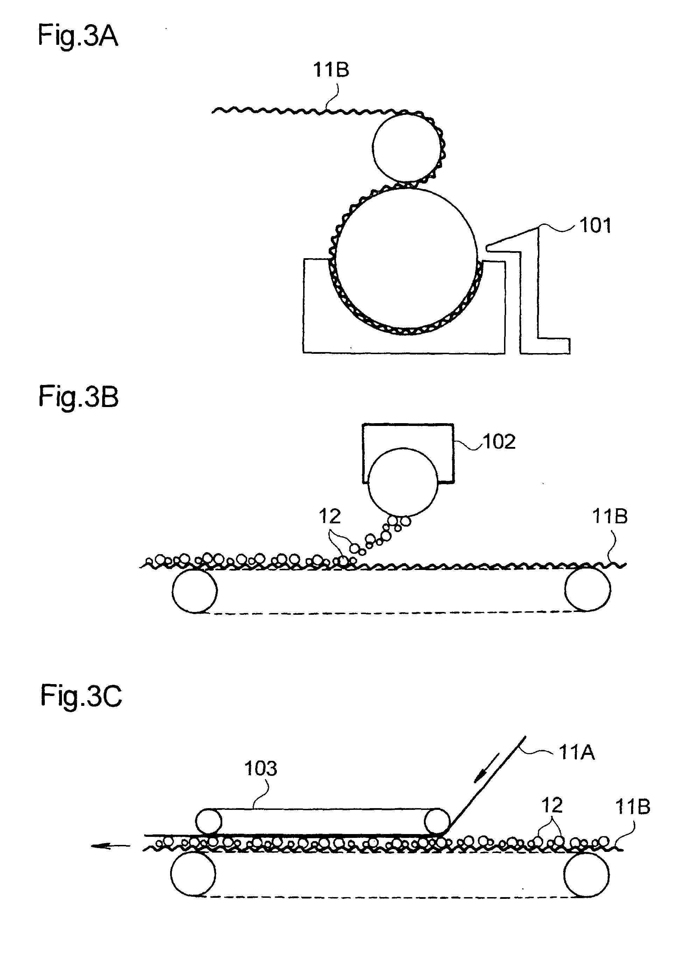

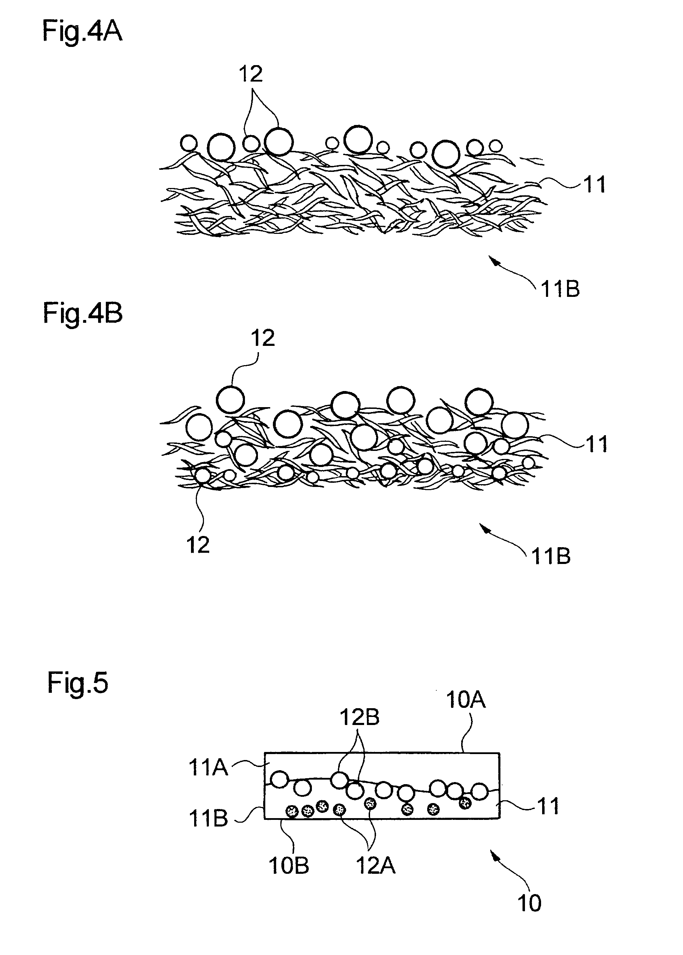

An example of a preferred process of producing the absorbent sheet 10 incorporating the first embodiment will now be described by referring to Figs. 3A to 3C and Fig. 4A and 4B.

The process of producing the absorbent sheet 10 includes a wet papermaking step, a dewatering step, a first drying step, a polymer sprinkling step, a vibrating and diffusing step, a wetting step, a laminating step, and a second drying step.

As illustrated in Fig. 3A, a lower fiber layer 11B is formed in a forming part 101 of a wet paper machine in the papermaking step, dewatered in the dewatering step, and sent to the first drying step where it is dried. The means for drying is not particularly limited and includes a Yankee dryer and a through-air dryer.

The dried lower fiber layer 11B is forwarded in the polymer sprinkling step where, as illustrated in Fig. 3B, polymer particles 12 having a constant size distribution are sprinkled from a hopper 102 over the upper surface of the lower fiber layer 11B so as to result in the structure illustrated in Fig. 4A.

The size distribution of the polymer particles 12 fed from the hopper 102 is measured, for example, as follows. The term "particle size" as used for the polymer particles 12 to be sprinkled denotes the diameter of a spherical particle 12 or the maximum sieve opening through which an irregular particle 12 is able to pass in sieve analysis. In sieve analysis, the polymer particles 12 are size classified using test sieves with 106 $\mu$m, 150 $\mu$m, and 500 $\mu$m openings, respectively, available from Tokyo Screen Co., Ltd. into four size fractions: -106 $\mu$m, +106/-150 $\mu$m, +150/-500 $\mu$m, and +500 $\mu$m, and the size distribution of the four fractions is represented by weight fraction. Where necessary, the size distribution may be analyzed more precisely by additionally using test sieves with 250 $\mu$m and 355 $\mu$m openings, respectively.

The lower fiber layer 11B with the polymer particles 12 on is given a vibration in the vibrating and diffusing step. The vibration occurs while the lower fiber layer 11B passes through press rolls or uneven surface rolls, or the vibration is produced by setting an uneven surface roll underneath the conveyer belt. While being vibrated, the polymer particles 12 are diffusively moved toward the second surface 11B to be embedded inside the lower fiber layer 11B as illustrated in Fig. 4B. Smaller polymer particles 12 are embedded deeper in the lower fiber layer 11B. Some of the large particles 12 remain on the upper surface of the lower fiber layer 11 B.

The lower fiber layer 11B is forwarded to the wetting step where it is sprayed with water and then to the laminating step.

In the laminating step, a separately prepared upper fiber layer 11A made up of a dry fibrous material 11 is superposed on the lower fiber layer 11B and joined together to form a laminate while moving in a pressing part 103 as shown in Fig. 3C. Because the polymer particles 12 remaining on the upper surface of the lower fiber layer 11B have developed tack on being wetted, the two fiber layers 11 A and 11B are bonded to each other via the tacky polymer particles 12.

The resulting laminate enters the second drying step where it is dried and lightly pressed to obtain the absorbent sheet 10 of the first embodiment as a unitary laminate.

In application of the absorbent sheet 10 of the first embodiment, if part of the body fluid once absorbed by the polymer particles 12 near the second surface 10B is released out of the polymer particles due to the adsorption equilibrium, it is prevented from vaporizing from the first surface 10A, i.e., the absorbing surface of the absorbent sheet 10. This is because the released fluid or water vapor generated therefrom will be re-absorbed by the upper polymer particles 12 or fibrous material 11 while moving up the distance to the first surface 10A.

The water vapor released from the polymer particles 12 near the second surface 10B escapes outside of the absorbent sheet 10 mostly from the side of the second surface 10B. In application to a sanitary napkin, for instance, the absorbent sheet 10 of the first embodiment can be disposed directly on a moisture permeable backsheet with its second surface 10B in contact with the backsheet. In such a configuration, the released water vapor escapes from the sanitary napkin primarily through the backsheet and is thereby prevented from causing discomfort, such as stuffiness or stickiness, while the napkin is in use.

Second to fourth preferred embodiments of the absorbent sheet according to the present invention will be described with reference to Figs. 5 through 10. The description on the first embodiment applies to the second to fourth embodiments unless otherwise specified. Elements in Figs. 5 to 10 that are identified with the same numerals as in Figs. 1 to 4 may be identical and will not be redundantly described.

As shown in Fig. 5, the absorbent sheet 10 incorporating the second preferred embodiment of the present invention contains two kinds of superabsorbent polymer particles different in rate of absorption, designated polymer particles 12A and polymer particles 12B. The polymer particles 12A having a higher absorption rate have a smaller average particle size than the polymer particles 12B having a lower absorption rate.

When the lower fiber layer 11B of the absorbent sheet 10 of the second embodiment is divided into regions by a plane perpendicular to the thickness direction of the sheet, the average particle size of the superabsorbent polymer 12 dispersed in each region decreases from the side of the first surface 10A to the side of the second surface 10B of the absorbent sheet 10. The region nearer to the second surface 10B contains more polymer particles 12A. The region nearer to the first surface 10A contains more polymer particles 12B.

The polymer particles 12A having a higher absorption rate preferably have a rate of absorbing a 0.9% edible salt aqueous solution (hereinafter simply referred to as an absorption rate) of 0.8 g/g/sec or higher, more preferably 1.0 g/g/sec or higher, as measured in accordance with JIS K 7224. On the other hand, the polymer particles 128 having a lower absorption rate preferably have an absorption rate of 0.7 g/g/sec or lower, more preferably 0.65 g/g/sec or lower. In order to draw liquid to near the second surface 10B and to fix the liquid there, it is desirable for the polymer particles 12A to have an absorption rate of at least 0.8 g/g/sec. If the polymer particles 12B have a higher absorption rate than the polymer particles 12A, a body liquid would be absorbed and fixed by the polymer particles 12B mostly dispersed in the region nearest to the first surface 10A before diffusing to the side of the second surface 10B.

In the whole region of the lower fiber layer 11B, the average particle size of the polymer particles 12A is preferably 100 to 300 $\mu$m, more preferably 100 to 250 $\mu$m, and that of the polymer particles 12B is preferably 250 to 500 $\mu$m, more preferably 250 to 350 $\mu$m.

When the lower fiber layer 11B is divided into, e.g., three equal regions by two planes perpendicular to the thickness direction, the proportion of the polymer particles 12B present in the region nearest to the first surface 10A is preferably 20% to 50% by weight based on all the polymer particles 12 in the absorbent sheet 10, while the proportion of the polymer particles 12A present in the region nearest to the second surface 10B is preferably 30% to 70% by weight on the same basis. The percent by weight as used herein with respect to the amount of the polymer particles is on a dry basis.

The polymer particles 12A also have a high swelling ratio (when simulated for artificial blood), while the polymer particles 12B have a low swelling ratio. Specifically, the swelling ratio of the polymer particles 12A in physiological saline is preferably 10 to 50 times, and that of the polymer particles 12B is preferably 5 to 30 times.

The absorbent sheet 10 of the second embodiment is otherwise structurally the same as in the first embodiment.

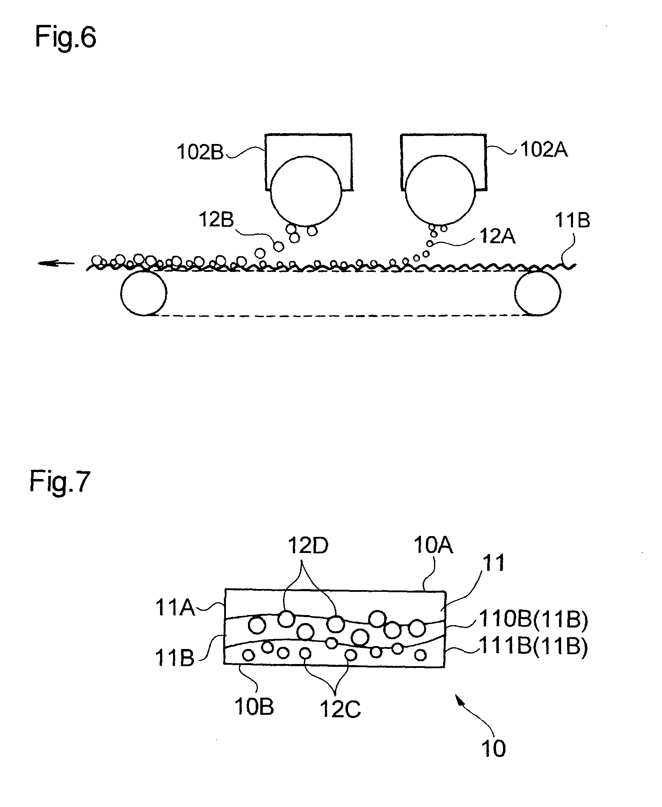

An example of a preferred process of producing the absorbent sheet 10 according to the second embodiment is described by way of Fig. 6.

The process of producing the absorbent sheet 10 of the second embodiment is the same as the process of producing the absorbent sheet of the first embodiment, except for the polymer sprinkling step as will be described below.

In the production of the absorbent sheet of the second embodiment, the polymer sprinkling step is carried out using two hoppers 102A and 102B as shown in Fig. 6. From the hopper 102A are fed polymer particles 12A having a higher absorption rate and a smaller particle size. From the hopper 102B are fed polymer particles 12B having a lower absorption rate and a greater particle size.

A lower fiber layer 11B that has been dried in the first drying step as described is conveyed to the polymer sprinkling step, where the polymer particles 12A from the hopper 102A are first sprinkled on the upper surface of the lower fiber layer 11B, and the polymer particles 12B from the hopper 102B are then sprinkled thereon. The lower fiber layer 11B having the polymer particles 12 thereon is subjected to the vibrating and diffusing step described. The vibrating and diffusing step may be provided between the two hoppers.

Containing two kinds of polymer particles 12A and 12B different in average particle size, absorption rate, and swelling ratio, the absorbent sheet 10 of the second embodiment has an advantage that the lower fiber layer 11B exhibits enhanced abilities to draw a body liquid toward the side of the second surface 10B and to fix the liquid there as well as the advantages of the first embodiment.

An absorbent sheet 10 incorporating the third preferred embodiment has a first lower fiber sublayer 110B and a second lower fiber sublayer 111 B superposed on the lower side of the first lower fiber sublayer 110B to form a lower fiber layer 11B as illustrated in Fig. 7.

The weight per unit area of each of the first lower fiber sublayer 110B and the second lower fiber sublayer 111B is in the same range as the lower fiber layer 11B in the first embodiment. That is, the lower fiber layer 11B in the third embodiment has about twice as much weight per unit area as that in the first embodiment. Therefore, the absorbent sheet 10 has an increased absorption capacity in its portion nearer to the second surface 10B.

The second lower fiber sublayer 111B positioned on the side of the second surface 10B contains superabsorbent polymer particles 12C, while the first lower fiber sublayer 110B positioned on the side of the first surface 10A contains superabsorbent polymer particles 12D. The polymer particles 12C have a smaller particle size than the polymer particles 12D. The polymer particles 12C and 12D may be the same or different in chemical and physical properties other than the size.

The fibrous material 11 in the first lower fiber sublayer 110B and that in the second lower fiber sublayer 111B preferably contain the above-described hydrophilic fiber, heat fusible fiber, and wet strength additive.

The composition of the fibrous material 11 of the first lower fiber sublayer 110B and that of the second lower fiber sublayer 111B may be the same or different. For example, the second lower fiber sublayer 111 B may be designed to have increased capillarity by using hydrophilic fiber the fineness of which is lower than that of the hydrophilic fiber used in the first lower fiber sublayer 110B.

The absorbent sheet 10 of the third embodiment is otherwise structurally the same as described with respect to the first embodiment.

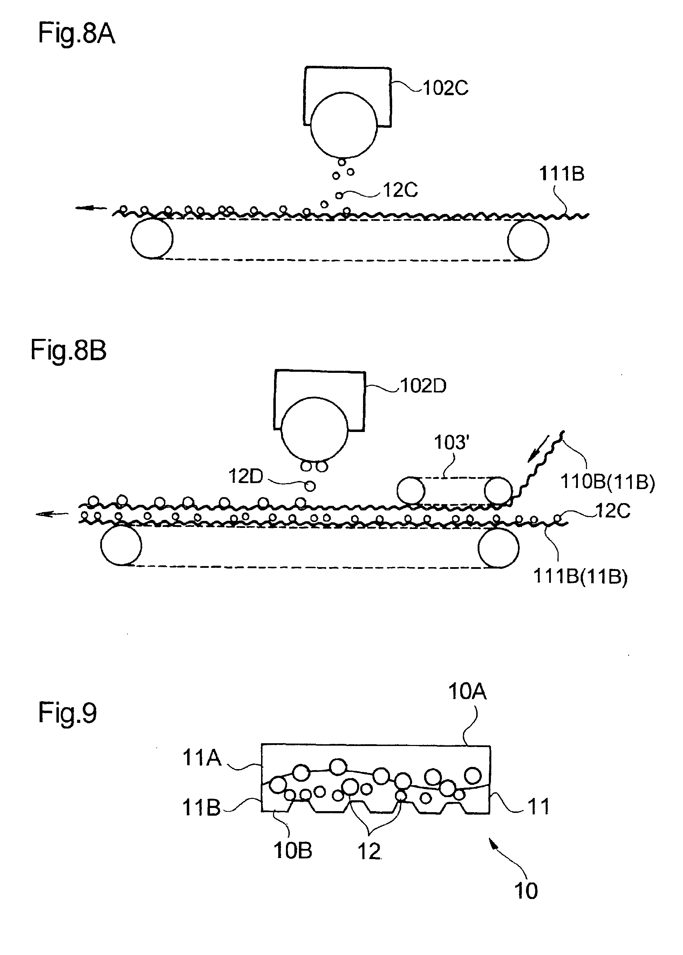

An example of a preferred process of producing the absorbent sheet 10 of the third embodiment is described by way of Figs. 8A and 8B.

The process of producing the absorbent sheet 10 of the third embodiment is the same as the process of producing the absorbent sheet of the first embodiment, except for the polymer sprinkling step as will be described below.

In the production of the absorbent sheet 10 according to the third embodiment, the polymer sprinkling step is divided into a first polymer sprinkling substep (Fig. 8A) and a second polymer sprinkling substep (Fig. 8B). The polymer particles 12C fed from a hopper 102C in the first polymer sprinkling substep have a smaller particle size than the polymer particles 12D fed from a hopper 102D in the second polymer sprinkling substep.

A second lower fiber sublayer 111B that has been dried in the first drying step described supra is transferred to the first polymer sprinkling substep, where smaller polymer particles 12C from the hopper 102C are sprinkled over the upper surface of the second lower fiber sublayer 111B as shown in Fig. 8A. The second lower fiber sublayer 111B having the polymer particles 12C thereon is further transferred to the second polymer sprinkling substep.

In the second polymer sprinkling substep, a separately prepared first lower fiber sublayer 110B is superposed on the polymer-sprinkled side of the second lower fiber sublayer 111B, and the stack of the two sublayers is pressed in a pressing part 103' into a laminate structure. The larger polymer particles 12D fed from the hopper 102D are sprinkled on the upper surface of the first lower fiber sublayer 110B of the laminate structure. The laminate structure is then forwarded to the vibrating and diffusing step described supra. Some of the polymer particles 12D move toward the second surface 10B by the vibration and are embeddedly disposed in the second lower fiber sublayer 111B.

In the third embodiment, since smaller polymer particles are dispersed in the side nearer to the second surface 10B in the polymer sprinkling step, it is easier than in the first and second embodiments to form the structure as designed to enable drawing an absorbed liquid toward the second surface 10B and fixing the drawn liquid there. The third embodiment produces the same effects and advantages as in the first embodiment as well.

An absorbent sheet 10 incorporating a fourth preferred embodiment of the present invention has the fibrous material 11 on the side of the second surface 10B densified in the thickness direction by heat embossing as shown in Fig. 9. More specifically, the lower fiber layer 11B is densified by heat embossing from the side of the second surface 10B to have a reduced fiber-to-fiber distance.

In more detail, densification of the fibrous material 11 results in an increase of the number of the fine voids defined by the interstices between fibers, which results in increased capillarity. The heat embossing provides enhanced unity between the upper and lower fiber layers 11A and 11B. Held by the thus densified fiber material 11, the polymer particles 12 dispersed in the lower fiber layer 11B are prevented from falling off more effectively.

It is preferred that the lower fiber layer 11B contain 1% to 60% by weight, more preferably 5% to 30% by weight, of heat fusible fibers to form the densified structure and to ensure unity of the upper and lower fiber layers 11A and 11B.

The absorbent sheet 10 of the fourth embodiment is otherwise structurally the same as that of the first embodiment.

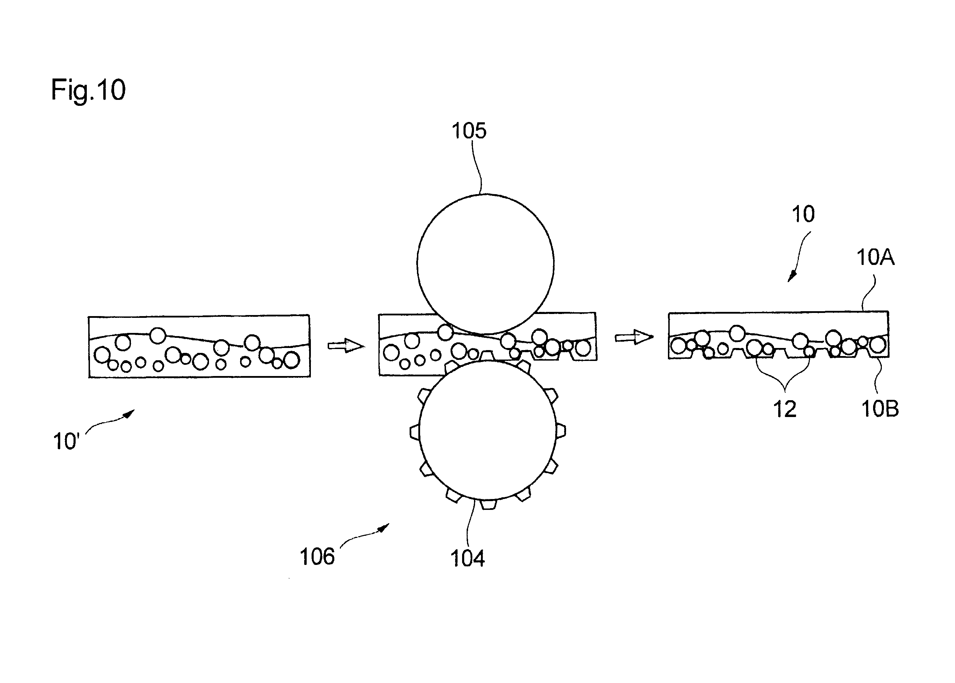

An example of a preferred process of producing the absorbent sheet 10 according to the fourth embodiment is described by way of Fig. 10.

The process of producing the absorbent sheet 10 of the fourth embodiment is the same as the process of producing the absorbent sheet of the first embodiment, except for further including a heat embossing step after the second drying step.

The heat embossing step is carried out as follows. An absorbent sheet 10' having been dried in the second drying step described supra is forwarded to the heat embossing step, where the absorbent sheet 10' is passed through an embosser 106. As illustrated in Fig. 10, the embosser 106 has an embossing roll 104 with a regular pattern engraved on its peripheral surface and an anvil roll 105 placed above the embossing roll 104, between which the absorbent sheet 10' runs with its upper fiber layer 11A facing up, i.e., in contact with the anvil roll 105.

The embossing roll 104 is configured to be heated by a heater, while the anvil roll 105 is configured to be cooled with cooling water so as not to raise its temperature. The embosser 6 is thus designed to heat only the lower fiber layer 11B of the absorbent sheet 10' to a prescribed temperature.

Heated and pressed in the heat embossing step, the lower fiber layer 11B of the absorbent sheet 10 has the fibrous material 11 densified. On the other hand, the upper fiber layer 11A, which has received only pressure and has been once compressed in the heat embossing step, returns to its state before the heat embossing after passing through the embosser 106.

The heat embossing step may be conducted before the polymer sprinkling step. In this case, the heat embossing is done to only the lower fiber layer 11B to densify the lower fiber layer 11B.

The absorbent sheet 10 of the fourth embodiment exhibits increased capability of drawing a liquid to the lower fiber layer 11B owing to the heat-embossing. The heat embossing also enhances the unity between the upper and lower fiber layers 11A and 11B, hardly leaving gaps between the two fiber layers where a liquid may stay. The absorbent sheet 10 of the present embodiment has the advantage of the first embodiment as well.

The absorbent sheet according to the present invention is not limited to the aforementioned embodiments; neither is it limited by the process of production. Various changes and modifications can be made to the aforementioned embodiments and processes of production without departing from the spirit and scope of the present invention.

The absorbent sheet of the present invention is applicable to, for example, sanitary napkins, panty liners, and incontinence pads.

With respect to particulars that have not been described for an embodiment, the corresponding description of other embodiments appropriately apply, and the particulars characteristic of an embodiment apply to other embodiments appropriately. Likewise, the particulars of any of the above-described processes of production that have not been described are complemented by the corresponding description of other processes, and the particulars characteristic of a process apply to other processes appropriately.

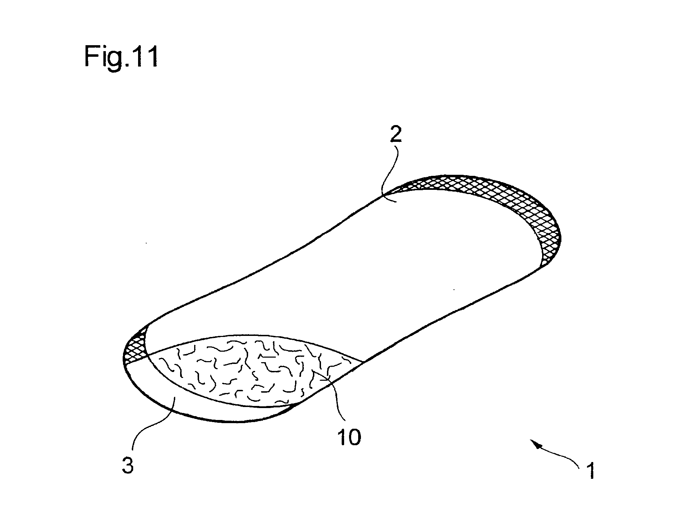

A preferred embodiment of the absorbent article according to the present invention, in which the absorbent sheet of the present invention is used, will be described by way of Fig. 11. The description given above with respect to the absorbent sheet 10 appropriately applies to the absorbent article unless otherwise specified. Elements in Fig. 11 that are identified with the same numerals as in Figs. 1 to 10 may be identical.

A sanitary napkin 1 shown in Fig. 11, a preferred embodiment of the absorbent article of the invention, is longer than is wide and has a liquid permeable topsheet 2, a liquid impermeable backsheet 3, and an absorbent sheet 10 interposed between the two sheets.

The backsheet 3 used in the present embodiment has moisture permeability.

Each of the topsheet 2 and the backsheet 3 has a length and a width with its length coinciding with the length of the sanitary napkin 1.

The absorbent sheet 10 is the above-described one. The absorbent sheet 10 is disposed with its first surface 10A facing the topsheet 2 and its second surface 10B facing the backsheet 3. The absorbent sheet 10 and the backsheet 3 are partly bonded to each other via a hot-melt adhesive applied to the second surface 10B of the absorbent sheet 10 in a dot or line pattern.

As the moisture permeable as well as liquid impermeable backsheet, a sheet having a great number of fine pores and therefore exhibiting high water vapor permeability can be used. Exemplary examples of such a backsheet include the one obtained by uniaxially or biaxially stretching a film of polyethylene having dispersed therein inorganic particles of calcium carbonate, barium sulfate, etc. or particles of an incompatible organic polymer, such as nylon or polystyrene. An example of such a moisture permeable and liquid impermeable film is one obtained by preparing a polyethylene film containing 30% to 60% by weight of calcium carbonate particles with an average particle size of 0.5 to 1.0 $\mu$m and weighing 20 to 50 g/m$^2$ and longitudinally stretching the film to double its length and to reduce the weight to 13 to 28 g/m$^2$.

A water repellent nonwoven fabric or a composite sheet of the above-described moisture permeable sheet and a water repellent nonwoven fabric is also useful as the moisture permeable backsheet.

The moisture permeable backsheet preferably has a water vapor transmission rate of 0.7 g/100 cm$^2$·hr or higher, more preferably 1.0 to 4.0 g/100 cm$^2$·hr. When the water vapor transmission rate of the backsheet is less than 0.7g/100cm$^2$·hr, the backsheet can be a barrier against water vapor release to the outside of the sanitary napkin 1.

A body fluid discharged on the sanitary napkin 1 passes through the topsheet 2 and, upon reaching the first surface 10A of the absorbent sheet 10, the liquid swiftly migrates toward the second surface 10B by the strong liquid drawing force of the lower fiber layer 11B. The liquid is absorbed by the polymer particles evenly distributed over the planes perpendicular to the thickness direction of the absorbent sheet 10 near the second surface 10B and fixed there.

Even if a body fluid once absorbed by the polymer particles 12 near the second surface 10B is partially released out of the polymer particles 12 due to the adsorption equilibrium, the released fluid or water vapor generated therefrom will be re-absorbed by the upper polymer particles 12 or fibrous material 11 while moving up because there is a distance to the first surface 10A. Vaporization of the absorbed body fluid from the first surface 10A is thus prevented.

The water vapor released from the polymer particles 12 near the second surface 10B escapes from the sanitary napkin 1 primarily through the moisture permeable backsheet 3 and is thereby prevented from causing discomfort, such as stuffiness or stickiness, while the napkin is in use.

As shown in Fig. 11, the topsheet 2 and the backsheet 3 extend outwardly from the longitudinal edges of the absorbent sheet 10 and joined together at the extensions by any known means, such as heat sealing or application of an adhesive.

On the garment facing side of the sanitary napkin 1 is applied a garment attachment adhesive for joining the garment facing side to the wearer's undergarment. The garment attachment adhesive is protected with a release sheet until use. The garment attachment adhesive is applied to the garment facing side of the backsheet 3 preferably in a dot or spiral pattern so as not to be a barrier against moisture permeation through the backsheet 3.

The sanitary napkin 1 of the present embodiment fixes most of the body fluid absorbed in the absorbent sheet 10 in the portion near the backsheet. Therefore, water vapor generated from the absorbed body fluid is made to escape through the moisture permeable backsheet without causing a wearer a discomfort due to stuffiness or stickiness.

The absorbent article according to the present invention is not limited to the above-described embodiment, and various modifications can be made therein without departing from the spirit and scope of the present invention.

Examples of the absorbent article of the present invention include not only sanitary napkins as in the above embodiment but also panty liners and incontinence pads.

The absorbent sheet of the invention and the absorbent article using the absorbent sheet hardly cause stuffiness and give a comfort to a wearer even after a large amount of a body fluid is discharged or after the absorbent article is worn for an extended period of time as well as exhibit high absorptivity and excellent resistance to leakage.

**Claims**

1. An absorbent sheet comprising a fibrous material and a particulate superabsorbent polymer and having a first surface and a second surface, wherein the absorbent sheet comprises two or more layers (11A, 11B) comprising the fibrous material,
   wherein the upper layer (11A) has a first surface (10A) that serves as a skin facing surface of the absorbent sheet, and wherein the lower layer (11B) forms the second surface (10B) of the absorbent sheet,
   the particulate superabsorbent polymer being dispersed, in the thickness direction of the absorbent sheet in a state of being held by the fibrous material such that,
   when the portion in the thickness direction of the absorbent sheet where the superabsorbent polymer is distributed is divided into regions by at least one plane perpendicular to the thickness direction of the absorbent sheet, the average particle size of the superabsorbent polymer in each region decreases from the region that is the nearest to the first surface (10A) to the region that is the nearest to the second surface (10B), and
   the number of interstices between the fibers and the particles increases in the direction from the first surface (10A) to the second surface (10B).

2. The absorbent sheet according to claim 1, wherein the superabsorbent polymer is dispersed in a plane perpendicular to the thickness direction of the sheet in the vicinity of the second surface.

3. The absorbent sheet according to claim 1 or 2, wherein the particulate superabsorbent polymer comprises two kinds different in absorption rate, the polymer having a higher absorption rate having a smaller average particle size than the polymer having a lower absorption rate.

4. The absorbent sheet according to any one of claims 1 to 3, having the fibrous material on the side of the second surface densified by heat embossing from that side.

5. An absorbent article comprising a topsheet, a backsheet, and the absorbent sheet according to any one of claims 1 to 4, the absorbent sheet being between the topsheet and the backsheet.

6. The absorbent article according to claim 5, wherein the backsheet has moisture permeability.

7. A process of producing the absorbent sheet of claim 1, comprising the steps of drying a first fiber layer formed by a wet papermaking technique, sprinkling superabsorbent polymer particles having a particle size distribution on the dried first fiber layer, giving vibration to the first fiber layer with the polymer particles thereon to diffuse the polymer particles in the thickness direction of the layer, and integrally laminating a second fiber layer on the polymer-sprinkled side of the first fiber layer.

**Patentansprüche**

1. Absorbierende Lage mit einem Fasermaterial und einem partikelförmigen superabsorbierenden Polymer und mit einer ersten Oberfläche und einer zweiten Oberfläche, wobei die absorbierende Lage zwei oder mehr Schichten (11A, 11B) mit dem Fasermaterial aufweist,
   wobei die obere Schicht (11A) eine erste Oberfläche (10A) aufweist, die als der Haut zugewandte Oberfläche der absorbierenden Lage dient, und wobei die untere Schicht (11B) die zweite Oberfläche (10B) der absorbierenden Lage bildet,
   wobei das partikelförmige superabsorbierende Polymer in der Dickenrichtung der absorbierenden Lage verteilt ist und dadurch durch das Fasermaterial so gehalten wird, dass, wenn der Abschnitt in der Dickenrichtung der absorbierenden Lage, in dem das superabsorbierende Polymer verteilt ist, durch zumindest eine senkrecht zur Dickenrichtung der absorbierenden Lage verlaufende Ebene in Bereiche geteilt ist, die durchschnittliche Teilchengröße des superabsorbierenden Polymers in jedem Bereich vom Bereich, der der ersten Oberfläche (10A) am nächsten ist, zum Bereich, der der zweiten Oberfläche (10B) am nächsten ist, abnimmt und
   die Zahl der Zwischenräume zwischen den Fasern und den Teilchen in Richtung von der ersten Oberfläche (10A) zur zweiten Oberfläche (10B) zunimmt.

2. Absorbierende Lage nach Anspruch 1, wobei das superabsorbierende Polymer in einer zur Dickenrichtung der Lage senkrechten Ebene nahe der zweiten Oberfläche verteilt ist.

3. Absorbierende Lage nach Anspruch 1 oder 2, wobei das partikelförmige superabsorbierende Polymer zwei Arten umfasst, die sich in der Absorptionsgeschwindigkeit unterscheiden, wobei das Polymer mit einer höheren Absorptionsgeschwindigkeit eine kleinere durchschnittliche Teilchengröße aufweist als das Polymer mit einer geringeren Absorptionsgeschwindigkeit.

4. Absorbierende Lage nach einem der Ansprüche 1 bis 3, wobei sich das Fasermaterial auf der Seite der zweiten Oberfläche befindet, die durch Heißprägen von dieser Seite aus verdichtet wurde.

5. Absorbierender Artikel mit einer Deckschicht, einer Rückschicht und der absorbierenden Lage nach einem der Ansprüche 1 bis 4, wobei sich die absorbierende Lage zwischen der Deck- und der Rückschicht befindet.

6. Absorbierender Artikel nach Anspruch 5, wobei die Rückschicht feuchtigkeitsdurchlässig ist.

7. Verfahren zur Herstellung der absorbierenden Lage nach Anspruch 1, mit den Schritten:

Trocknen einer mit Hilfe eines Papiererzeugungs-Nassverfahrens gebildeten ersten Faserschicht, Besprenkeln der getrockneten ersten Faserschicht mit superabsorbierenden Polymerpartikeln mit einer Teilchengrößenverteilung, wobei die erste Faserschicht mit den darauf verteilten Polymerpartikeln in Vibration versetzt wird, um die Polymerpartikel in der Dickenrichtung der Schicht zu zerstreuen, und Auflaminieren einer zweiten Faserschicht auf die mit Polymerpartikeln besprenkelte Seite der ersten Faserschicht.

## Revendications

1. Feuille absorbante comprenant un matériau fibreux et un polymère super-absorbant particulaire et ayant une première surface et une seconde surface, dans laquelle la feuille absorbante comprend deux couches ou plus (11A, 11B) comprenant le matériau fibreux,
dans laquelle la couche supérieure (11A) a une première surface (10A) qui sert de surface faisant face à la peau de la feuille absorbante, et dans laquelle la couche inférieure (11B) forme la seconde surface (10B) de la feuille absorbante,
le polymère super-absorbant particulaire étant dispersé, dans le sens de l'épaisseur de la feuille absorbante, dans un état où il est maintenu par le matériau fibreux, de sorte que,
quand la portion, dans le sens de l'épaisseur de la feuille absorbante où le polymère super-absorbant est réparti, est divisée en zones par au moins un plan perpendiculaire au sens de l'épaisseur de la feuille absorbante, la taille moyenne de particules du polymère super-absorbant dans chaque zone diminue, de la zone qui est la plus proche de la première surface (10A) à la zone qui est la plus proche de la seconde surface (10B) et
le nombre d'interstices entre les fibres et les particules augmente dans la direction allant de la première surface (10A) vers la seconde surface (10B)

2. Feuille absorbante selon la revendication 1, dans laquelle le polymère super-absorbant est dispersé dans un plan perpendiculaire au sens de l'épaisseur de la feuille, à proximité de la seconde surface.

3. Feuille absorbante selon les revendications 1 ou 2, dans laquelle le polymère super-absorbant particulaire comprend deux types différents en matière de taux d'absorption, le polymère doté d'un taux d'absorption supérieur ayant une taille de particules moyenne inférieure au polymère doté d'un taux d'absorption inférieur.

4. Feuille absorbante selon l'une quelconque des revendications 1 à 3, dans laquelle le matériau fibreux sur le côté de la seconde surface est densifié par un emboutissage à chaud de ce côté.

5. Article absorbant comprenant une feuille supérieure, une feuille de support et la feuille absorbante selon l'une quelconque des revendications 1 à 4, la feuille absorbante étant comprise entre la feuille supérieure et la feuille de support.

6. Article absorbant selon la revendication 5, dans lequel la feuille de support est perméable à l'humidité.

7. Procédé de production de la feuille absorbante selon la revendication 1, comprenant les étapes consistant à sécher une première couche de fibres formée par une technique de fabrication de papier humide, à pulvériser les particules de polymère super-absorbant ayant une distribution de taille de particules sur la première couche de fibres séchée, à appliquer des vibrations à la première couche de fibres, sur laquelle se trouvent les particules de polymères, afin de diffuser les particules de polymère dans le sens de l'épaisseur de la couche, et à laminer intégralement une seconde couche de fibres sur le côté vaporisé par le polymère de la première couche de fibres.

Fig.1

Fig.2

## Fig.3A

## Fig.3B

## Fig.3C

Fig.4A

12

11

11B

Fig.4B

12

11

12

11B

Fig.5

12B

10A

11A

11

11B

10B

12A

10

## Fig.6

## Fig.7

Fig.8A

Fig.8B

Fig.9

Fig.10

EP 1 800 638 B1

Fig.11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7132126 A

- JP 8246395 A